# EUROPEAN PATENT APPLICATION

(11) **EP 4 681 671 A1**
(43) Date of publication of application: **21.01.2026**
(21) Application number: 25190130.2
(22) Date of filing: 17.07.2025
(51) Int. Cl.: A61B 18/18

(54) **MICROWAVE ABLATION PROBE CHOKE**

(30) Priority: 19.07.2024 US 202418778190
(71) Applicant: Varian Medical Systems, Inc., Palo Alto, CA 94304 (US)
(72) Inventor: MAY, Justin, Austin, 78738 (US); ANDERSON, David, Chanhassen, 55317 (US); GOMEZ, Virgilio, Kyle, 78640 (US); CHAMANI, Faraz, Dallas, 75235 (US)
(74) Representative: Mathisen & Macara LLP

(57) **Abstract**

A microwave ablation probe (200) includes an outer shell (302) defining an inner cavity axially extending to a probe tip (304), a cable (306) extending in the inner cavity and comprising an antenna (312), and a choke (308) coupled to the cable in the inner cavity. The choke (308) comprising an inner layer (404) mechanically locked to an outer casing (402).

## Description

### TECHNICAL FIELD

The disclosure relates to chokes used in microwave ablation probes. More specifically, the disclosure relates to chokes used in microwave ablation probes that may comprise one or more interlocking features to minimize or prevent relative movement between the elements of the choke.

### BACKGROUND

Microwave ablation probes can be used in clinical treatments such as thermal ablation treatments. In such treatments, thermal ablation can be used to destroy undesirable tissue such as malignant cells in a body. A microwave ablation antenna can be included in the probe and be used to deliver Radio Frequency (RF) energy such as microwave energy to a target tissue to heat the target tissue and destroy the target tissue. The microwave ablation antenna can be positioned inside the ablation probe that can position the microwave ablation antenna proximate the target tissue.

In some treatments, the ablation probe is guided to the target tissue though other tissue or near to tissues or body structures that it is desirable not to damage during treatment. It is desirable, therefore, to maintain a small size of the microwave ablation antenna and/or the needle of the probe. In this manner, damage to tissues and other body structures that may be located close to the target tissue to be destroyed is minimized or prevented. It is also desirable that the microwave ablation antenna produce a repeatable and known ablation or heating zone relative to the position of the microwave ablation antenna. The heating zone can then be reliably delivered to the target tissue without damage or with minimized damage to surrounding tissue and body structures. There exists a need, therefore, for improved microwave ablation probes or needle designs that are sufficiently small while also delivering known and repeatable ablation zones.

### SUMMARY

The present disclosure is directed to microwave ablation probes that may include a choke positioned on a power cable inside the needle of the probe adjacent to the antenna. The choke may include an inner layer that is mechanically locked to the outer conductive casing, said inner layer may be an inner insulating layer. The mechanical lock may prevent or minimize movement of the inner insulating layer relative to the outer casing. Such a choke is an improvement over existing chokes that may experience relative movement or deformation between the inner insulating layer and the outer conductive layer of the choke during assembly of the microwave ablation probe.

The present invention provides a microwave ablation probe in accordance with the appended claims. In accordance with some embodiments, a microwave ablation probe is provided. The microwave ablation probe may include an outer shell defining an inner cavity axially extending to a probe tip, a cable extending in the inner cavity and comprising an antenna, and a choke coupled to the cable in the inner cavity, the choke comprising an inner layer mechanically locked to an outer casing. The ablation probe may include a handle and/or a needle. The needles may comprise any suitable material, such as metal, fiberglass, composite, alloy or other rigid material. The needle suitably is a cylindrical body and pointed tip. The needle may be elongated in a longitudinal or axial direction. The various elements of the needle may be concentrically positioned about the longitudinal axis thereof. The needle is suitably hollow to allow other components to be positioned inside. For example, a power line, an antenna, and/or coolant lines may be located inside the hollow needle. The ablation probe may further comprise one or more sensors, such as one or more temperature sensors, thermocouples and/or flow sensors. The ablation probe may further comprise a supply cable, which may be an elongated tube or conduit. One or more of a power line, to deliver a power signal from a microwave generator to the microwave antenna, a coolant supply line and a coolant return line and one or more wires to provide signals from the one or more sensors may extend within an outer shell of the supply cable. The supply cable may be any suitable length, such as at least 3 meters (10 feet) or at least 3.65 meters (12 feet). The needle may include the shell, a tip, a power cable, the choke and a coolant lumen. The shell may be the outermost member of the needle. The shell may be a hollow cylindrical tube that defines an inner cavity. The tip is suitably positioned at the distal end of the shell. The shell may have a first portion made of a rigid material and a second portion located at or near a distal end of a non-conductive material. The second portion may be located at a portion of the needle at or near the antenna. The antenna may be located at or near the tip. The power cable may be a coaxial cable and may include an outer conductor and an inner conductor. The cable may be positioned as the innermost element of the needle, for example the cable may be positioned along the longitudinal axis of the needle. The antenna may be positioned at a distal end of the cable at or near the tip of the needle. The antenna can be configured in any suitable manner such as monopole antenna or a dipole antenna. A coolant lumen can be located radially outward of the cable and radially inward of the shell. The coolant limen suitably extends longitudinally into the needle along the central axis, with a terminating end position at or near the antenna.

The choke may be a cylindrically shaped member surrounding the cable. The choke may comprise an outer casing and an inner layer. The outer casing may be shaped as a cylindrical tube of material, suitably of conductive material. In one aspect, the outer casing of the choke is made of a conductive material and the inner layer is made of an insulating material. The conductive material suitably provides a conductive outer casing, which conductive outer casing may be electrically coupled to the outer conductor of the power cable. The choke may be positioned radially outward of the power cable and radially inward of a coolant lumen. The choke may be positioned on an axial side of the antenna away from a tip of the probe. The choke may include a mechanical lock that locks the relative position between the outer casing and the inner layer.

In another aspect, the outer casing may include a cylindrical wall and at least one lock opening positioned through the cylindrical wall.

In another aspect, the inner layer may be positioned radially inward of the outer casing and protrude into the at least one lock opening.

In another aspect, the outer casing may include a cylindrical wall and at least two lock openings positioned through the cylindrical wall.

In another aspect, the inner layer may be positioned radially inward of the outer casing and protrudes into each of the at least two lock openings.

In another aspect, a first lock opening of the at least two lock openings may be positioned radially opposite a second lock opening of the at least two lock openings.

In another aspect, the outer casing may extend between a connecting end at which the outer casing is coupled to the cable and a locking end opposite to the connecting end. The connecting end may be the end at which the choke is electrically coupled to the outer conductor of the cable. The at least one lock openings may be positioned at or near the locking end.

In another aspect, the inner layer may be molded into the outer casing.

In another aspect, the inner layer may radially protrude into at least one lock in the outer casing. The inner layer may include a first post and, optionally, a second post formed of the inner layer material that protrudes into the first lock opening and, optionally, the second lock opening. The first post and, optionally, the second post may be oriented substantially perpendicular to the axis of the choke.

In another aspect, the at least one lock may include a circular opening.

In some embodiments of the present disclosure, a choke for use in a microwave ablation probe may include an outer conductive casing and an inner insulating layer that radially protrudes into at least one lock opening in the outer conductive casing.

In one aspect, the outer conductive casing may include a cylindrical tube extending along an axis between a connecting end and a locking end and the inner insulating layer may be positioned radially inward of outer conductive casing.

In another aspect, the at least one lock opening may be positioned at or near the locking end of the outer conductive casing.

In another aspect, the connecting end of the outer conductive casing may be configured to electrically couple to an outer conductor of a microwave ablation power cable.

In another aspect, a center line of the at least one lock opening is oriented substantially perpendicular to the axis of the outer conductive casing.

In another aspect, the inner insulating layer defines a cylindrical cavity extending along the axis to allow a microwave ablation power cable to extend therethrough.

In some embodiments of the present disclosure, a microwave ablation apparatus according to the appended claims is provided. The microwave ablation apparatus may include an ablation console comprising a microwave generator; and a microwave ablation probe coupled to the ablation console. The microwave generator may provide a power signal to an antenna in the ablation probe, suitably via a supply cable extending from the console to the probe. The supply cable may also provide a coolant to the ablation probe. The coolant may be a saline solution or other coolant that may be supplied to the ablation probe from a coolant receptacle via a pump cartridge or other pump mechanism. The microwave ablation probe may include an outer shell defining an inner cavity axially extending to a probe tip, a cable extending in the inner cavity and comprising an antenna, and a choke coupled to the cable in the inner cavity. The choke may include an inner layer mechanically locked to an outer casing.

In some embodiments of the present disclosure, a method of producing a choke for a microwave ablation probe according to the appended claims is provided. The method may include providing a cylindrical tube comprising at least one lock opening extending through a wall of the tube, and depositing a layer of insulating material along an inner surface of the cylindrical tube such that the insulating material protrudes into the at least one lock opening.

In one aspect, the layer of insulating material may be deposited in a liquid form and allowed to solidify.

In another aspect, the step of depositing the layer of insulating material may be performed in a mold.

Further areas of applicability will become apparent from the description provided herein. The description and specific examples in this summary are intended for purposes of illustration only and are not intended to limit the scope of the present disclosure.

### BRIEF DESCRIPTION OF THE DRAWINGS

The features and advantages of the present disclosures will be more fully disclosed in, or rendered apparent by the following detailed descriptions of example embodiments. The detailed descriptions of the example embodiments are to be considered together with the accompanying drawings wherein like numbers refer to like parts and further wherein:
FIG. 1 is an isometric view of an example microwave ablation apparatus in accordance with some embodiments of the present disclosure.
FIG. 2 is an example microwave ablation probe that may include a choke in accordance with some embodiments of the present disclosure
FIG. 3 is a side sectional view of an example needle of the microwave ablation probes of the present disclosure.
FIG. 4 is isometric view of a choke in accordance with some embodiments of the present disclosure.
FIG. 5 is a sectional view of the choke of FIG. 4.

### DETAILED DESCRIPTION

The description of the preferred embodiments is intended to be read in connection with the accompanying drawings, which are to be considered part of the entire written description of these disclosures. While the present disclosure is susceptible to various modifications and alternative forms, specific embodiments are shown by way of example in the drawings and will be described in detail herein. The objectives and advantages of the claimed subject matter will become more apparent from the following detailed description of these exemplary embodiments in connection with the accompanying drawings.

It should be understood, however, that the present disclosure is not intended to be limited to the particular forms disclosed. Rather, the present disclosure covers all modifications, equivalents, and alternatives that fall within the spirit and scope of these exemplary embodiments. The terms "couple," "coupled," "operatively coupled," "operatively connected," and the like should be broadly understood to refer to connecting devices or components together either mechanically, electrically, wired, wirelessly, or otherwise, such that the connection allows the pertinent devices or components to operate (e.g., communicate) with each other as intended by virtue of that relationship.

In the present disclosure the singular forms "a," "an," and "the" include the plural reference, and reference to a particular numerical value includes at least that particular value, unless the context clearly indicates otherwise. When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive. Where present, all ranges are inclusive and combinable. For example, when a range of "1 to 5" is recited, the recited range should be construed as including ranges "1 to 4", "1 to 3", "1-2", "1-2 & 4-5", "1-3 & 5", "2-5", and the like. In addition, when a list of alternatives is positively provided, such listing can be interpreted to mean that any of the alternatives may be excluded, e.g., by a negative limitation in the claims. For example, when a range of " 1 to 5" is recited, the recited range may be construed as including situations whereby any of 1, 2, 3, 4, or 5 are negatively excluded; thus, a recitation of "1 to 5" may be construed as "1 and 3-5, but not 2", or simply "wherein 2 is not included." It is intended that any component, element, attribute, or step that is positively recited herein may be explicitly excluded in the claims, whether such components, elements, attributes, or steps are listed as alternatives or whether they are recited in isolation.

The microwave ablation probes of the present disclosure may include a choke that is included in the needle to control a size and shape of the ablation zone produced by the microwave ablation antenna. The chokes of the present disclosure may include an outer casing and an inner insulating layer. The inner insulating layer may be locked into position relative to the outer casing to prevent and/or minimize relative movement between the outer casing and the insulating layer. By locking the elements of the choke relative to each, the size and shape of the ablation zone is known after the choke is installed in the microwave ablation probe.

It has been observed, for example, that during the manufacture, use, of the ablation probe, that the insulating layer may move or deform relative to initial or desired position of the insulating layer relative to the outer casing. Movement and/or deformation of the insulating layer may impact or change the size and/or shape of the ablation zone. In some examples, the insulating layer is mechanically locked into a desired position relative to the outer casing by depositing insulating material into an opening or other feature of the outer casing to lock the insulating layer into the desired position. The microwave ablation probes and the included chokes of the present disclosure are improvement over existing designs because the microwave ablation probes and included chokes of the present disclosure may produce known, repeatable ablation zones.

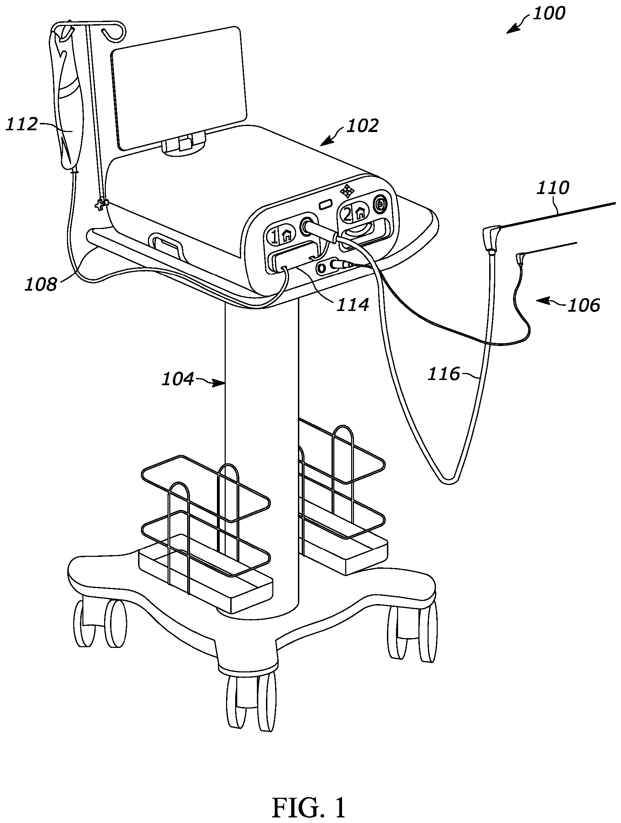

Turning now to FIG. 1, an example microwave ablation apparatus 100 is shown. In this example, the ablation apparatus 100 includes an ablation console 102, and an ablation probe 110. The ablation console 102 may be portable, in some examples, and may be positioned on a platform 108 of stand 104 to allow the console 102 to be easily moved and positioned as desired during treatment. The console 102 may include a microwave generator that may provide a power signal that is sent to an antenna in the needle of the ablation probe 110. The power signal may be provided to the antenna via a supply cable 116 that may extend from the console 102 to the ablation probe 110. The supply cable 116 may also provide a coolant to the ablation probe 110. The coolant may be a saline solution or other coolant that may be supplied to the ablation probe 110 from a coolant receptacle 112 via a pump cartridge 114 or other pump mechanism.

During an ablation treatment, the needle of the ablation probe 110 may be positioned at or near a target tissue in a patient. A power signal may be supplied to the ablation probe 110 from the console 102. The power signal may be supplied to the ablation probe 110 via the supply cable 116. The power signal may cause microwaves to be emitted from an antenna in the needle of the ablation probe 110. The microwaves may cause the tissue at the needle to be heated to a suitable temperature to destroy the target tissue. A temperature sensor 106 may be positioned at or near the target tissue to measure a temperature that may be achieved at or near the ablation zone. In addition, and as further described below, the needle or the handle of the ablation probe 110 may include other thermocouples or temperature probes to provide information regarding temperatures of the coolant or the needle.

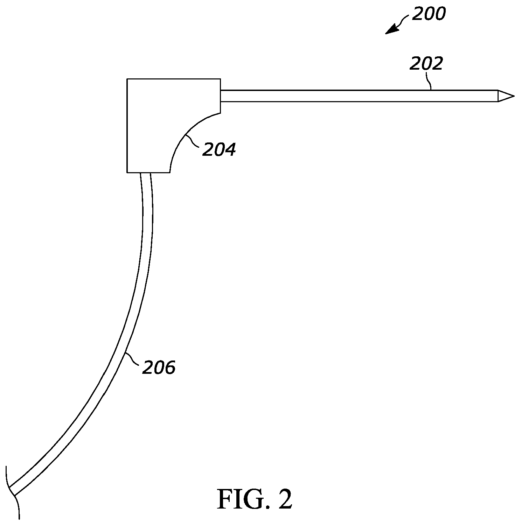

Referring now to FIG. 2, an example ablation probe 200 is shown. The ablation probe 200 may be used with the microwave ablation apparatus 100 previously described. The ablation probe 200 may include a needle 202, a handle 204, and a supply cable 206. The ablation probe 200 may be a disposable or single-use probe that is used during a single procedure or for a single patient. In other examples, the ablation probe 200 (or portions of the ablation probe 200) may be re-used.

The needle 202 of the ablation probe 200 may be an elongated member that maybe inserted into a patient at or near the target tissue. The needle 202 may be a metal, fiberglass, composite, alloy or other rigid material. The needle 202 may be cylindrical and pointed. The needle 202 may be hollow such that various elements are positioned inside the needle 202. For example, a power line and an antenna may be located inside needle 202. The power line may be positioned inside the needle 202 to deliver a power signal from a microwave generator to the antenna. Coolant lines may also be positioned inside the needle to deliver coolant to a distal end of the needle 202. The coolant may be used to control a temperature of the needle 202 and/or to control a size and shape of the ablation zone that may be created at the distal end of the needle 202.

The handle 204 may connect and/or operably couple aspects of the ablation probe 200 that are provided from the supply cable 206 to the needle 202. The supply cable 206 may include a power line that delivers the power signal from the microwave generator. The supply cable 206 may also include a coolant supply line and a coolant return line. The coolant supply line may deliver coolant to the needle 202 and the coolant return line may convey the coolant away from the needle 202 after it has been used to cool the needle 202. The supply cable 206 may also include one or more wires that may be provide signals from one or more temperature sensors, thermocouples, flow sensors, or other sensors that may provide information regarding operating characteristics of the ablation probe 200.

The supply cable 206 may be an elongated tube or conduit and each of the power line, the coolant supply line, the coolant return line, and other wires and lines may extend within an outer shell of the supply cable 206. A length of the supply cable 206 may allow a user of the ablation probe 200 to position the needle 202 of the ablation probe 200 as desired and to allow the patient to be moved in and/or out of an imaging device during a treatment. For example, a location of the needle 202 may be determined using CT, MRI, Ultrasound, or other imaging devices before, during, or after treatment is performed. Thus, the supply cable 206 may be sufficiently long to allow these diagnostic procedures to be performed. In some examples, the supply cable 206 is at least 3.65 meters (12 feet) in length. In other examples, the supply cable 206 is at least 3 meters (10 feet) in length. In other examples, other lengths may be used.

The handle 204 may connect and/or operably couple the supply cable 206 to the needle 202. The power signal, the coolant, and/or sensor signals may be conveyed or delivered from the supply cable 206 to the needle 202 through the handle 204. The handle 204 may include a housing that may enclose various aspects such as fluid connections, electrical connections, electrical components, and the like. The handle 204 may be configured to orient the needle 202 in a desired alignment relative to the longitudinal axis of the supply cable 206. In the example shown, the handle 204 is a right-angle handle. The handle 204 orients the needle 202 at about 90 degrees (or substantially perpendicular) to the longitudinal axis of the supply cable 206. In other examples, the handle 204 may orient the needle 202 at a different angle relative to the longitudinal axis of the supply cable 206.

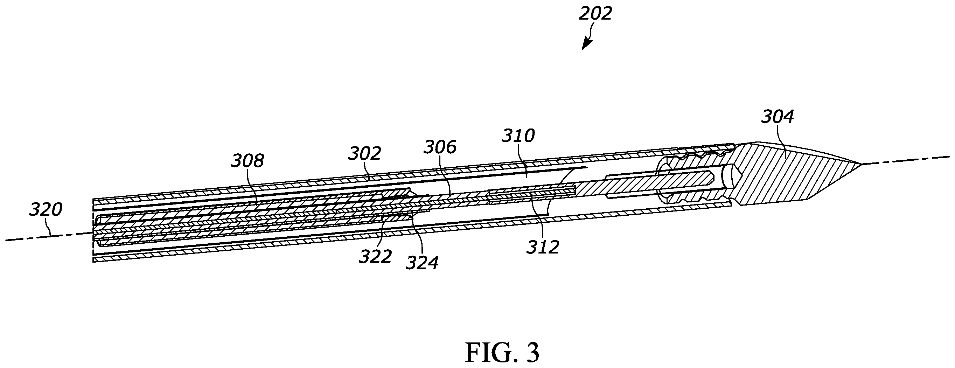

Turning now to FIG. 3, an example needle 202 is shown. The needle 202 may include a shell 302, a tip 304, a power cable 306, a choke 308, and a coolant lumen 310. During a treatment, a microwave power signal may travel to an antenna 312 along the power cable 306 from a microwave generator. The power cable 306 may be a coaxial cable that can include an outer conductor and an inner conductor. The current that is passed to the antenna 312 may bounce back or be reflected back (in a direction away from the tip 304) along the outer conductor of the power cable 306. This back current is undesirable because the back-current may cause the ablation zone that is created at the needle 202 to become elongated or have a tear-drop shape rather than being more spherical in shape. The elongated ablation zone may radiate or heat healthy tissues adjacent or near the target tissues. It is desirable, therefore, to prevent and/or minimize distortions that may occur to the size and/or shape of the ablation zone due to reflected to back-current.

The choke 308 may be provided in the needle 202 to prevent, reduce, or minimize back-current that travels back along the power cable 306 in a direction away from the tip 304. The choke 308, as further described below, may include a conductive outer casing that can be electrically coupled to the outer conductor of the power cable 306 to limit, reduce, and/or minimize current that is reflected back along an outer surface of the power cable 306 away from the antenna 312.

The needle 202 may be elongated in a longitudinal or axial direction along axis 320. The various elements of the needle 202 may be concentrically positioned about the axis 320. The shell 302 may be the outermost member of the needle 202 and may be a hollow cylindrical tube that defines an inner cavity. The outer surface of the shell 302 may have a smooth profile to facilitate insertion of the needle 202 into a patient at the target tissue. The tip 304 may be positioned at the distal end of the shell 302 and may be pointed to further facilitate insertion and positioning of the needle 202.

The shell 302 can be made of multiple materials joined together. A first portion of the shell 302 may be made of a stainless steel or other relatively rigid material to provide structure to the needle 202. A second portion located at or near a distal end of the shell may be made of a non-conductive material such as a plastic, fiberglass, ceramic, composite or other non-metallic material. The second portion is made of suitable non-conductive material so as to allow RF energy to be conveyed from the antenna 312 through the shell 302. The end portion may, therefore, be located at a portion of the needle 202 at or near the antenna 312 which is typically located at or near the tip 304.

The cable 306 may be positioned as the innermost element of the needle 202 and may be positioned in a center of the needle along the axis 320. The cable 306 is configured to deliver a suitable current from a microwave generator (not shown) to cause microwave energy (or other RF energy) to be emitted from the antenna 312. The cable 306 can be a coaxial cable that includes a center conductor and an outer conductor. The center conductor may be positioned at a central axis of the cable 306. The outer conductor may be separated from the inner conductor and be configured as a cylinder of conductive material positioned radially outward of the inner conductor along the length of the cable 306.

The antenna 312 may be positioned at a distal end of the cable 306 at or near the tip 304 of the needle 202. The antenna 312 can be configured in any suitable manner such as monopole antenna or a dipole antenna. While one type or configuration of the antenna 312 may be shown in the figures, it should be appreciated that other types or configurations can also be used. As previously described, the antenna 312 is configured to emit RF or microwave energy to heat tissue that is located at or near the antenna 312. In this manner, the needle 202 can generate an ablation zone that is generally located around the antenna 312 at or near the tip 304 of the needle 202.

When in use, the energy of the antenna 312 and current reflected along the outer conductor of the cable 306 may cause the needle 202 to increase in temperature. The heating of the needle 202 may cause the heating zone to become elongated in an axial or longitudinal direction of the needle 202. The elongated heating zone may have an elliptical or teardrop shape, for example. In addition, if the exterior surface of the shell 302 increases in temperature, the tissue in contact with the shell 302 may stick to the shell 302. When the needle 202 is retracted after treatment, the tissue may tear resulting in tearing and/or bleeding that is undesirable. To prevent undesirable heating of the needle 202 and/or to maintain a more spherical ablation zone shape, the needle 202 may include a cooling system to actively cool the needle 202 during operation.

In this example, the needle 202 includes the coolant lumen 310. The coolant lumen 310 can be located between the cable 306 and the shell 302. Thus, the coolant lumen 310 can be located radially outward of the cable 306 and radially inward of the shell 302. The coolant lumen 310 extends longitudinally into the needle 202 along the central axis 320 with a terminating end of the coolant lumen 310 being positioned at or near the antenna 312.

The coolant lumen 310 is configured to deliver a cooling fluid toward the tip 304 of the needle 202. While not shown, the cooling system can include a source of cooling fluid and/or a suitable pump that can deliver a flow of cooling fluid in the interior of the coolant lumen 310 toward the tip 304 of the needle 202. The cooling fluid may return to the source of the cooling fluid or be exhausted or otherwise deposited to another location. During operation, the cooling fluid can flow in a flow path in which the cooling fluid is delivered to the needle in an input channel defined by an interior surface of the cooling tube and an exterior surface of the cable 306. The cooling fluid may flow away from the tip 304 in a return channel defined by an exterior or outer surface of the coolant lumen 310 and an interior surface of the shell 302. The cooling fluid may be any suitable flowable cooling liquid or gas such as water, saline, carbon dioxide gas, or the like.

The choke 308 may be positioned in the shell 302 of the needle 202 in a position radially outward of the power cable 306 and radially inward of the coolant lumen 310. The choke 308 may be configured as cylindrically shaped member that surrounds the cable 306. The choke 308 may be electrically coupled to an outer conductor of the cable 306. The choke 308 may be coupled at an axial position on the cable 306 at or near the antenna 312. The choke 308 may be positioned on an axial side of the antenna 312 away from the tip 304. The choke 308 may be coupled to the cable 306 using a solder joint 324 at the connecting end 322 of the choke 308.

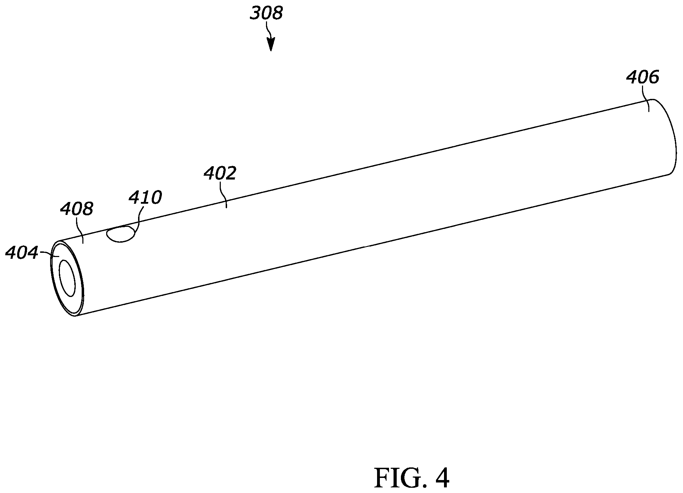

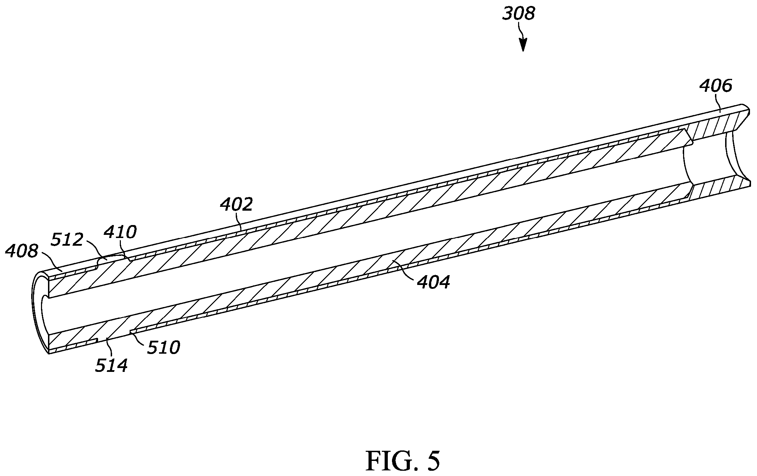

Referring now to FIGs. 4 and 5, an example choke 308 is shown. The choke 308 may include an outer casing 402 and an inner layer 404. The outer casing 402 may be shaped as a cylindrical tube of material. The outer casing 402 may be formed from a cylindrical wall of conductive material such as stainless steel, copper, or other metal or alloy. The outer casing 402 may extend axially from a locking end 408 to a connecting end 406. The connecting end 406 may be the end at which the choke 308 is electrically coupled to the outer conductor of the cable 306.

The inner layer 404 may be a layer of material that is positioned radially inward of the outer casing 402. The inner layer 404 may be made of a different material as the outer casing 402. The inner layer 404 may be made of an insulating material such as a polymer, perylene, polytetrafluoroethylene (PTFE), polyimide, epoxy, or the like. The insulating inner layer 404 may separate the outer casing from the cable 306 except at the connecting end 406 where the choke 308 is coupled to the cable 306.

It was observed that during the assembly of the needle 202 that the inner layer 404 could move or become deformed from its original position or desired position relative to the outer casing 402. For example, it was observed that during the soldering process that deposits a small amount of solder to electrically join the connecting end 406 of the choke 308 to the cable 306, the inner layer 404 could become dislodged or become deformed such that the end of the inner layer 404 would extend past or protrude out from the locking end 408 of the outer casing 402. This change to the choke 308 caused a change to the size and shape of the ablation zone produced by the needle 202.

To prevent and/or minimize relative movement between the inner layer 404 and the outer casing 402, the choke 308 includes a mechanical lock that locks the relative position between the outer casing 402 and the inner layer 404. In the example shown, the mechanical lock between the elements is accomplished using two lock openings that extend through the wall of the outer casing 402. In this example, the outer casing 402 includes a first lock opening 410 and a second lock opening 510 (see FIG. 5). Each of the first lock opening 410 and the second lock opening 510 may be a circular hole that extends through the wall of the outer casing 402. The first lock opening 410 and the second lock opening 510 may be positioned on opposite radial sides of the outer casing 402.

The inner layer 404 may be molded into the outer casing 402. During such a process, liquid or formable material may be deposited into the outer casing 402 while the outer casing is positioned in a mold. The material may flow into the outer casing 402 to form the cylindrical inner layer 404. During such process, the flowable material may also flow into the first lock opening 410 and the second lock opening 510. The flowable material may be allowed to cool or otherwise solidify. When solid, the inner layer 404 may include a first post 512 and a second post 514 formed of the inner layer material that protrudes into the first lock opening 410 and the second lock opening 510. The first post 512 and the second post 514 may be oriented substantially perpendicular to the axis of the choke 308.

The first post 512 and the second post 514 may lock the inner layer 404 into the outer casing 402. As can be appreciated, since the inner layer 404 protrudes into the first lock opening 410 and the second lock opening 510, the inner layer 404 is locked into relative axial position relative to the outer casing.

In the example shown, the choke 308 includes two lock openings, namely first lock opening 410 and second lock opening 510. The first lock opening 410 and the second lock opening 510 are located at the locking end 408 of the outer casing 402. The first lock opening 410 and the second lock opening 510 are located radially opposite to one another on opposite sides of the outer casing 402. It should be appreciated, however, that in other examples, the outer casing 402 may include more than two lock openings. In still other examples, the outer casing may include one lock opening. In other examples, the outer casing may include a series of openings or an array of lock openings.

In various other examples, other mechanical locks may be used to lock the inner layer 404 relative to the outer casing 402. In other examples, lock slots may be used that can be similar to the lock openings previously described but may have a shape different from a circular or oval opening. In other examples, the inner surface of the outer casing may include grooves, slots, ribs, or other features that may create a discontinuous surface to which the inner layer 404 may lock during forming.

One or more methods of producing a choke are contemplated in the present disclosure. In one example, a method may include providing an outer casing for the production of the choke. The outer casing may be a cylindrical tubular member with at least one lock opening extending through the wall of the outer casing.

An insulating material may be deposited into the outer casing to form a cylindrical layer of the insulating material positioned radially inward of the outer casing. Such material may be deposited while the outer casing is located in a mold. The insulating material may be allowed to flow into the at least one lock opening. The insulating material may be allowed to cool and/or to solidify inside the outer casing. The choke formed from the outer casing and the inner layer of insulating material may be positioned over a power cable of a microwave ablation probe and soldered in a position adjacent the antenna.

The following is a list of non-limiting illustrative embodiments disclosed herein:
Illustrative embodiment 1: A microwave ablation probe comprising: an outer shell defining an inner cavity axially extending to a probe tip; a cable extending in the inner cavity and comprising an antenna; and a choke coupled to the cable in the inner cavity, the choke comprising an inner layer mechanically locked to an outer casing.
Illustrative embodiment 2: The microwave ablation probe of illustrative embodiment 1, wherein the outer casing of the choke is made of a conductive material and the inner layer is made of an insulating material.
Illustrative embodiment 3: The microwave ablation probe of any of illustrative embodiments 1 or 2, wherein the outer casing comprises a cylindrical wall and at least one lock opening positioned through the cylindrical wall.
Illustrative embodiment 4: The microwave ablation probe of illustrative embodiment 3, wherein the inner layer is positioned radially inward of the outer casing and protrudes into the at least one lock opening.
Illustrative embodiment 5: The microwave ablation probe of any of illustrative embodiments 1 to 4, wherein the outer casing comprises a cylindrical wall and at least two lock openings positioned through the cylindrical wall.
Illustrative embodiment 6: The microwave ablation probe of illustrative embodiment 5, wherein the inner layer is positioned radially inward of the outer casing and protrudes into each of the at least two lock openings.
Illustrative embodiment 7: The microwave ablation probe of illustrative embodiment 6, wherein a first lock opening of the at least two lock openings is positioned radially opposite a second lock opening of the at least two lock openings.
Illustrative embodiment 8: The microwave ablation probe of any of illustrative embodiments 1 to 7, wherein the outer casing extends between a connecting end at which the outer casing is coupled to the cable and a locking end opposite to the connecting end, the at least two lock openings being positioned at or near the locking end.
Illustrative embodiment 9: The microwave ablation probe of any of illustrative embodiments 1 to 8, wherein the inner layer is molded into the outer casing.
Illustrative embodiment 10: The microwave ablation probe of any of illustrative embodiments 1 to 9, wherein the inner layer radially protrudes into at least one lock in the outer casing.
Illustrative embodiment 11: The microwave ablation probe of illustrative embodiment 10, wherein the at least one lock comprises a circular opening.
Illustrative embodiment 12: A choke for use in a microwave ablation probe comprising an outer conductive casing and an inner insulating layer that radially protrudes into at least one lock opening in the outer conductive casing.
Illustrative embodiment 13: The choke of illustrative embodiment 12, wherein outer conductive casing comprises a cylindrical tube extending along an axis between a connecting end and a locking end and the inner insulating layer is positioned radially inward of outer conductive casing.
Illustrative embodiment 14: The choke of illustrative embodiment 13, wherein the at least one lock opening is positioned at or near the locking end of the outer conductive casing.
Illustrative embodiment 15: The choke of any of illustrative embodiments 13 or 14, wherein the connecting end of the outer conductive casing is configured to electrically couple to an outer conductor of a microwave ablation power cable.
Illustrative embodiment 16: The choke of any of illustrative embodiments 12 to 16, wherein a center line of the at least one lock opening is oriented substantially perpendicular to the axis of the outer conductive casing.
Illustrative embodiment 17: The choke of any of illustrative embodiments 12 to 16, wherein the inner insulating layer defines a cylindrical cavity extending along the axis to allow a microwave ablation power cable to extend therethrough.
Illustrative embodiment 18: A microwave ablation apparatus comprising: an ablation console comprising a microwave generator; and a microwave ablation probe coupled to the ablation console, the microwave ablation probe comprising: an outer shell defining an inner cavity axially extending to a probe tip; a cable extending in the inner cavity and comprising an antenna; and a choke coupled to the cable in the inner cavity, the choke comprising an inner layer mechanically locked to an outer casing.
Illustrative embodiment 19: A method of producing a choke for a microwave ablation probe comprising: providing a cylindrical tube comprising at least one lock opening extending through a wall of the tube; and depositing a layer of insulating material along an inner surface of the cylindrical tube such that the insulating material protrudes into the at least one lock opening.
Illustrative embodiment 20: The method of illustrative embodiment 19, wherein the layer of insulating material is deposited in a liquid form and allowed to solidify.
Illustrative embodiment 21: The method of any of illustrative embodiments 19 or 20, wherein the step of depositing the layer of insulating material is performed in a mold.

The foregoing is provided for purposes of illustrating, explaining, and describing embodiments of these disclosures. Modifications and adaptations to these embodiments will be apparent to those skilled in the art and may be made without departing from the scope or spirit of these disclosures.

## Claims

1. A microwave ablation probe (200) comprising:
an outer shell (302) defining an inner cavity axially extending to a probe tip (304);
a cable (306) extending in the inner cavity and comprising an antenna (312); and
a choke (308) coupled to the cable in the inner cavity, the choke comprising an inner layer (404) mechanically locked to an outer casing (402).

2. The microwave ablation probe of claim 1, wherein the outer casing (402) of the choke (308) is made of a conductive material and the inner layer (404) is made of an insulating material.

3. The microwave ablation probe of claim 1 or 2, wherein the outer casing (402) comprises a cylindrical wall and at least one lock opening (410, 510) positioned through the cylindrical wall and,
optionally, the inner layer (404) is positioned radially inward of the outer casing (402) and protrudes into the at least one lock opening (410, 510).

4. The microwave ablation probe of claim 1 or 2, wherein the outer casing (402) comprises a cylindrical wall and at least two lock openings (410, 510) positioned through the cylindrical wall and,
optionally, the inner layer (404) is positioned radially inward of the outer casing (402) and protrudes into each of the at least two lock openings (410, 510).

5. The microwave ablation probe of claim 4, wherein a first lock opening (410) of the at least two lock openings is positioned radially opposite a second lock opening (510) of the at least two lock openings.

6. The microwave ablation probe of claim 4 or 5, wherein the outer casing (402) extends between a connecting end (406) at which the outer casing is coupled to the cable and a locking end (408) opposite to the connecting end, the at least two lock openings (410, 510) being positioned at or near the locking end (408).

7. The microwave ablation probe of any one of the preceding claims, wherein the inner layer (404) is molded into the outer casing (402).

8. The microwave ablation probe of any one of the preceding claims, wherein the inner layer (404) radially protrudes into at least one lock in the outer casing and, optionally, the at least one lock comprises a circular opening.

9. A choke (308) for use in a microwave ablation probe comprising an outer conductive casing (402) and an inner insulating layer (404) that radially protrudes into at least one lock opening (410, 510) in the outer conductive casing.

10. The choke of claim 9, wherein outer conductive casing comprises a cylindrical tube extending along an axis (320) between a connecting end (406) and a locking end (408) and the inner insulating layer (404) is positioned radially inward of outer conductive casing (402) and,
optionally, the at least one lock opening (410, 510) is positioned at or near the locking end (408) of the outer conductive casing (402).

11. The choke of claim 10, wherein the connecting end (406) of the outer conductive casing is configured to electrically couple to an outer conductor of a microwave ablation power cable.

12. The choke of claim 10 or 11, wherein a center line of the at least one lock opening (410, 510) is oriented substantially perpendicular to the axis (320) of the outer conductive casing (406).

13. The choke of one of one claims 10-12, wherein the inner insulating layer (404) defines a cylindrical cavity extending along the axis (320) to allow a microwave ablation power cable (306) to extend therethrough.

14. A microwave ablation apparatus (100) comprising:
an ablation console (102) comprising a microwave generator; and
a microwave ablation probe (110, 200) coupled to the ablation console, the microwave ablation probe comprising:
an outer shell (302) defining an inner cavity axially extending to a probe tip (304);
a cable (306) extending in the inner cavity and comprising an antenna (312); and
a choke (308) coupled to the cable (306) in the inner cavity, the choke comprising an inner layer mechanically locked to an outer casing.

15. A method of producing a choke (308) for a microwave ablation probe (110, 200) comprising:
providing a cylindrical tube comprising at least one lock opening (410, 510) extending through a wall of the tube; and
depositing a layer of insulating material (404) along an inner surface of the cylindrical tube such that the insulating material protrudes into the at least one lock opening (410, 510) and,
optionally, the layer of insulating material is deposited in a liquid form and allowed to solidify in a mold.
